# EUROPEAN PATENT SPECIFICATION

(11) **EP 4 494 616 B1**
(45) Date of publication and mention of the grant of the patent: **12.08.2026**
(21) Application number: 24189762.8
(22) Date of filing: 19.07.2024
(51) Int. Cl.: A61F 13/00, A61F 15/00, A61F 17/00, A63H 3/36

(54) **ROLE PLAY FIRST AID KIT AND SIMULATED BANDAGE**
ROLLENSPIEL-ERSTHILFEKIT UND SIMULIERTE BANDAGE
KIT DE PREMIERS SECOURS POUR JEU DE RÔLE ET BANDAGE SIMULÉ

(30) Priority: 20.07.2023 US 202318224419
(43) Date of publication of application: 22.01.2025
(73) Proprietor: Melissa & Doug, LLC, Wilton, CT 06897 (US)
(72) Inventor: ABLES, Cristina, Riverview, FL 33569 (US); BUTLER, Jeffrey, Rye Brook, NY 10573 (US)
(74) Representative: Bugnion Genève

(56) References cited:
- US-A1- 2016 270 968
- US-A1- 2023 285 195

## Description

### BACKGROUND OF THE INVENTION

### Field of the Invention

The present disclosure relates to a simulated wound that can be removably applied to a user's skin to create the appearance of an actual wound, such as a cut, scrap, or abrasion. The present disclosure further relates to a simulated bandage that allows a user to pretend to treat a simulated wound on the user's skin. More particularly, the present disclosure relates to a patch that, when wetted, shows a simulated wound on the skin and to a simulated bandage that, when applied to the wetted patch, pulls the patch from the skin, creating the illusion that the wound has been healed. The present disclosure further relates to a kit including such patches, simulated bandages, and accessories related to administering first aid.

### Description of the Related Art

Children enjoy playing games where they have the sensation that they are acting as an adult. Toys that allow children to pretend to act as medical professionals are well known. Such toys may provide children with simulated medical instruments, such a syringes, stethoscopes, tweezers, medicine bottles, and the like. A child can manipulate these objects while pretending to be a doctor, nurse, or other health professional. Such toys allow a child to use his or her imagination, to envision a visit to the doctor or hospital, and to pretend to heal themselves and their playmates. Such toys may also help children facing actual health problems better cope with frightening medical experiences by enacting situations for themselves.

The experience of playing with toys that simulate medical procedures may be more engaging if the procedure seems more realistic. For example, a child may pretend that she or her playmate has a cut or scratch. The child may enjoy applying real bandages to herself or her playmates to pretend to treat the wound. Since there is no actual wound, the child must imagine it and image that the wound has healed when the bandage is removed.

There is a need for a toy that creates a realistic-looking simulated wound and that allows the child to pretend to treat that wound.

### BRIEF SUMMARY OF THE INVENTION

In a first aspect of the invention, there is provided a patch adapted to display a simulated wound, the patch as defined in claim 1, and further embodiments thereof are defined in dependent claims 2 to 7.

In a second aspect of the invention, there is provided a kit for creating a simulated wound, the kit as defined in claim 8 and a further embodiment thereof further defined in dependent claim 9.

In a third aspect of the invention, there is provided a simulated bandage as defined in claim 10, and further embodiments thereof defined in dependent claims 11 to 14.

In a fourth aspect of the invention, there is provided a method for creating a simulated wound, the method as defined in claim 15, and a further embodiment thereof defined in dependent claim 16.

Embodiments of the disclosure, not according to the claims but present for illustration purposes, provide a toy that includes a mechanism to simulate a skin wound, such as a scratch, abrasion, cut, insect bite or a suture. According to one aspect, such a wound is simulated by a patch of fabric that is opaque or nearly opaque when dry and that has a simulated wound printed on the fabric. The patch is applied to skin and saturated with water. The material forming the patch becomes translucent when wetted. The simulated wound and the skin beneath the patch become visible through the patch, creating the illusion that that there is an actual wound on the skin.

According to one aspect of the disclosure, not according to the claims but present for illustration purposes, the simulated wound is printed in colors that contrast with the skin to enhance the visual impact of the wound. According to another aspect, a plurality of patches are provided as part of a kit printed with a variety of simulated wounds. The user can select a particular simulated wound and imagine a scenario that logically connects with the wound selected. For example, the child may envision having fallen down and scraped her hand. The child might select a patch with a simulated abrasion. As part of her play, the child would apply the patch to her hand and wet the patch to create what looks like an abrasion.

According to another aspect of the disclosure, not according to claims but present for illustration purposes, the patch is thin and flexible to conform to the skin. Once the patch has been placed on the skin and wetted, capillary forces between the wetted patch and the skin hold the patch in contact with the skin. This temporarily fixes the patch to the skin, allowing the child to enact a play scenario involving the simulated wound.

According to another embodiment of the disclosure, not according to the claims but present for illustration purposes, a simulated bandage is provided along with one or more of the patches as described above. The simulated bandage has a top side that may be decorated to simulate an actual bandage, such as a Band-Aid^{®} adhesive bandage manufactured by Johnson & Johnson Consumer, Inc. One or more engaging regions are provided on the bottom side of the simulated bandage. According to one embodiment, the engaging regions are covered with flexible hooks, such as the hooks provided as part of a "hook-and-loop" fastener. According to this embodiment, the top surface of the patch is provided with loops, pores, or fibers that engage with the hooks when the bottom side of the simulated bandage is contacted with the top surface of the patch. Once the hooks engage with the patch, the bandage is pulled away from the skin. Because the patch is held to the skin only by capillary forces, the patch, along with the simulated wound lifts away from the skin easily, creating the illusion that the simulated wound has healed. According to one embodiment, the patch is unchanged by the process of adhering and removing from the skin. Such a patch can be reused repeatedly.

According to another embodiment of the disclosure, not according to claims but present for illustration purposes, a plurality of simulated bandages are provided as part of a kit along with one or more patches. Simulated bandages may be provided in a number of shapes and sizes to simulate the shapes and sizes of actual bandages and other first aid products. According to one aspect of the disclosure, simulated bandages are shaped as rectangles, squares, ovals, circles, stars, or any other shape that would simulate a bandage. According to another aspect, the bandage has an extended length to simulate a strip of gauze that can be wrapped around a part of a person's body to simulate a wound dressing.

According to one embodiment of the disclosure, not according to the claims but present for illustration purposes, the simulated bandage includes a fastening mechanism that releasably holds one portion of the bandage in connection with another portion of the same bandage. The fastening mechanism may comprise two components of a closure disposed at different locations on the bandage. When the bandage is wrapped around a body part, for example, a user's finger, the two components contact one another to hold the bandage onto the body part. According to one embodiment, the fastening mechanism comprises a hook-and-loop closure. According to a further embodiment, the hook component of the hook-and-loop closure also forms part of the engaging portion and functions to engage with the patch to pull it from the skin. According to another embodiment, the fastening mechanism comprises a magnet closure with a magnet disposed on one portion of the bandage and a ferromagnetic piece on another part of the bandage. Bringing these components together holds the two portions of the bandage together.

According to another embodiment of the disclosure, not according to the claims but present for illustration purposes, a water dispenser is provided to deliver water to the user's skin and to the patch to wet the patch as described above. The water dispenser may be a brush, a nozzle, a sponge, or a sprayer. According to one embodiment, the water dispenser is a container with a reservoir connected to a small opening. Water is delivered by dabbing the opening against the skin and the patch. The size and shape of the opening is selected to limit the flow of water from the reservoir. According to a further embodiment, the dispenser includes a removable cap that engages with the dispenser and closes the opening to keep water in the container when the container is not in use.

### BRIEF DESCRIPTION OF THE DRAWINGS

The above and/or other aspects of the disclosure will be more apparent by describing in detail exemplary embodiments of the disclosure with reference to the accompanying drawings, in which:
Figs. 1A, 1B, and 1C illustrate a patch for creating a simulated wound according to embodiments of the disclosure and the steps for applying the patch to the skin of a user to create the simulated wound;
Figs. 2A, 2B, and 2C show exemplary embodiments of patches, such as the one illustrated in Figs. 1A, 1B, and 1C, according to embodiments of the disclosure;
Fig. 3 shows a patch according to an embodiment of the disclosure applied to a user's skin;
Fig. 4 shows a dispenser pen according to an embodiment of the disclosure;
Fig. 5 shows a cap for the dispenser pen of Fig. 4 according to an embodiment of the disclosure;
Fig. 6 shows the cap of Fig. 5 connected with the dispenser pen of Fig. 4 ;
Figs. 7A and 7B show the top side and bottom side, respectively, of a simulated bandage according to an embodiment of the disclosure;
Fig. 8 show bandages according to embodiments of the disclosure with decorative images applied to top surfaces thereof;
Fig. 9 show a simulated bandage according to an embodiment of the disclosure;
Figs. 10A, 10B, and 10C show steps for applying a patch according to embodiments of the disclosure to a user's skin to create a simulated wound, for applying a simulated bandage according to an embodiment of the disclosure, and for removing the bandage and the patch from the skin;
Figs. 11A, 11B, 11C, 11D, and 11E show steps for applying a patch according to a further embodiment of the disclosure to a user's skin to create a simulated wound, for applying a simulated bandage according to a further embodiment of the disclosure, and for removing the bandage and the patch from the skin; and
Figs. 12A and 12B show a kit including a plurality of patches for creating simulated wounds, a plurality of simulated bandages, and a dispenser pen according to embodiments of the disclosure.

### DETAILED DESCRIPTION OF EXEMPLARY EMBODIMENTS

Exemplary embodiments of the disclosure will now be described below by reference to the attached Figures. The described exemplary embodiments are intended to assist the understanding of the invention and are not intended to limit the scope of the invention in any way. Like reference numerals refer to like elements throughout.

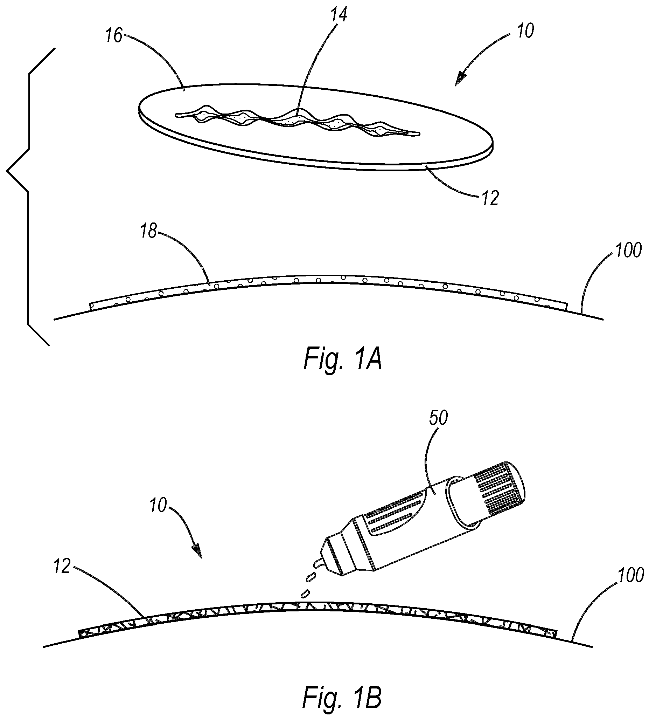

Fig. 1A shows a patch 10 according to embodiments of the disclosure. Patch 10 is formed by a fabric layer 12. A simulated wound image 14 is applied to the fabric layer. Fabric layer 12 is formed from a thin flexible material that can be wetted with water or other liquid. The material forming fabric layer 12 is selected to be relatively opaque when dry and that becomes translucent when wetted. According to one embodiment, fabric layer 12 is white or a neutral color. According to another embodiment, fabric layer 12 is formed from a material known to be safe for contact with human skin. According to one embodiment, fabric layer 12 is formed from a woven or non-woven textile, for example, a natural or synthetic textile such as a cotton or wool fabric, gauze, muslin, linen, or a synthetic material such as polyethylene, polypropylene or the like. According to a preferred embodiment, fabric layer 12 is formed from non-woven polypropylene. Fabric layer 12 is preferably thin and flexible to allow patch 10 to conform to curved surfaces, such as the skin on a person's limb. According to a preferred embodiment, fabric layer has a thickness of about 0.2 millimeters ("mm").

According to one embodiment, simulated wound image 14 is applied to fabric layer 12 by printing, such as screen printing, thermal transfer printing, hand drawing, or other technique for applying images to fabric or other sheets of material know to those of skill in the field of the disclosure.

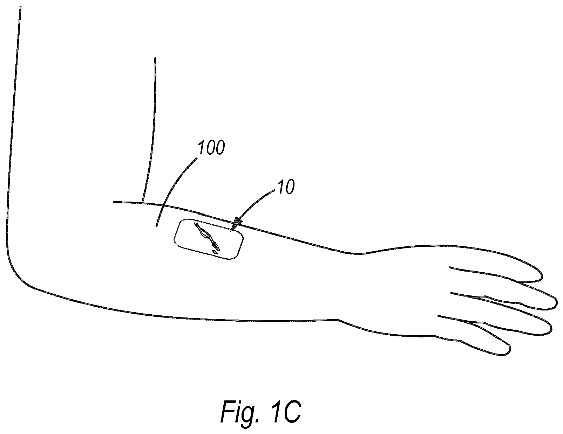

Figs. 1A, 1B, and 1C show use of patch 10 according to one embodiment of the disclosure. As shown in Fig. 1A, a layer of liquid 18 is applied to a user's skin 100. Liquid layer 18 may be applied by spraying, by brushing, by application using a fingertip or from an applicator, or by immersing the limb in water, for example, while bathing. Once the skin surface 100 is wetted, patch 10 is applied to the skin 100.

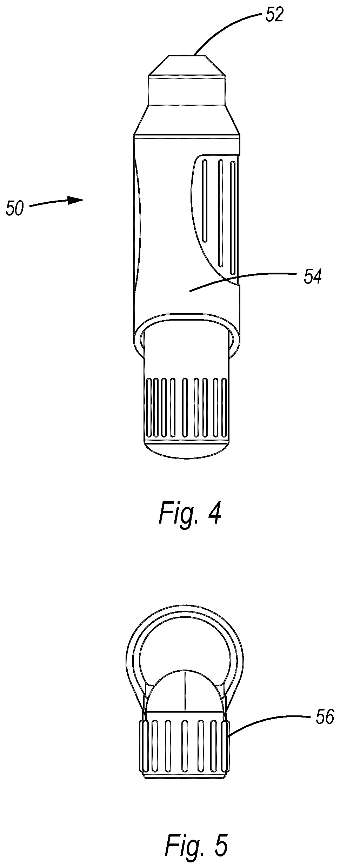

As shown in Fig. 1B, additional water may be applied to patch 10 to assure that it is fully saturated. This additional liquid, as well as liquid layer 18, shown in Fig. 1A, could be applied using dispenser pen 50, shown in Fig. 4 and described below. According to one embodiment, the liquid is water. According to other embodiments, other liquids may be used to saturate patch 10 within the scope of the disclosure. The liquid may be an aqueous solution, for example, sea water, or may include a dye solution to simulate blood associated with a simulated wound. The liquid may be a non-aqueous material, for example, mineral oil, petroleum jelly, baby oil, hand lotion, sunscreen, and the like that can safely be applied to a user's skin. According to further embodiments, the liquid may be a combination of liquids.

Fig. 1C shows patch 10 applied to a user's skin 100. Because liquid has permeated patch 10, fabric layer 12 becomes translucent so that the color of the user's skin 100 is at least partially visible through patch 10. As a result, simulated wound image 14 appears to be an actual wound on the skin 100. Surface tension of the liquid layer causes patch 10 to adhere to the skin in the same manner that wet clothing sticks to wet skin. This effect allows patch 10 to remain adhered to skin 100 as long as patch 10 remains saturated. According to one embodiment, the user can apply additional liquid to patch 10, such as shown in Fig. 1B, to maintain adhesion.

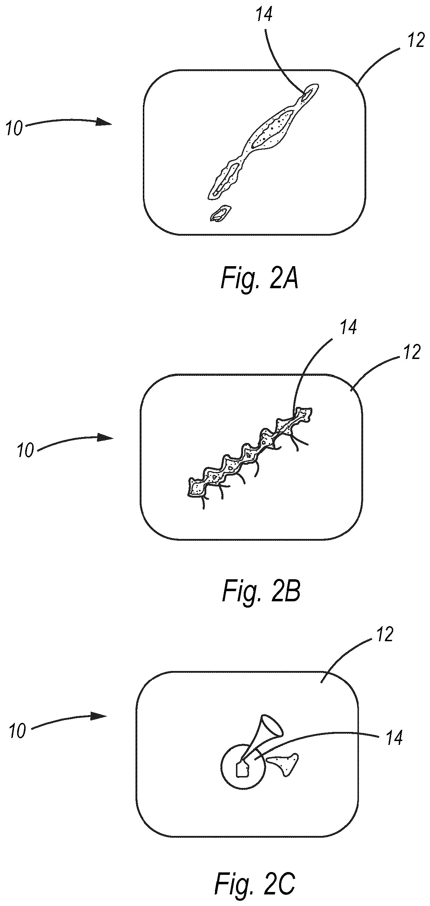

Figs. 2A to 2C show exemplary embodiments of patches 10 with different simulated wound images 14, for example, a cut or scratch ( Fig. 2A ), stiches ( Fig. 2B ), or a thorn puncture ( Fig. 2C ). These are exemplary images and are not intended to limit the scope of the disclosure. Other images might also be applied to patch 12, for example, of a simulated tattoo or body piercing, or of a different kind of wound.

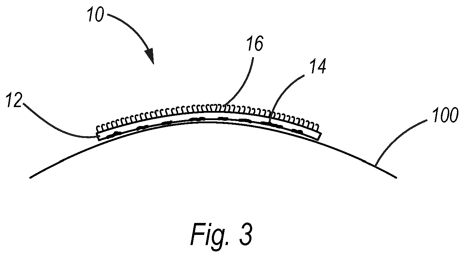

Fig. 3 shows a patch 10 according to a further embodiment of the disclosure. Patch 10 includes a gripping layer 16 on its upper surface. According to one embodiment, gripping layer 16 is formed by the material forming fabric layer 12. According to this embodiment, fabric layer 12 includes fibers that protrude from the upper surface of patch 10 that will facilitate the removal of the patch form the skin, as will be explained more fully below. According to another embodiment, gripping layer 16 is a layer of material, such as the material forming the "loop" portion or a "hook-and-loop" fastener. Gripping layer 16 is selected to be translucent when wetted so that it allows a user's skin to be seen below the patch surrounding the simulated wound image 14.

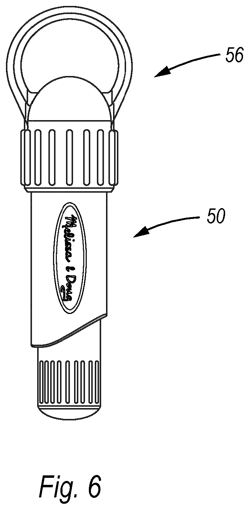

Fig. 4 shows dispenser pen 50 according to embodiments of the disclosure. Dispenser 50 includes a reservoir 54 for holding a quantity of water or other liquid. A dispensing orifice 52 is provided at one end of dispenser 50. The size of orifice 52 is selected so that liquid from reservoir 54 can flow outward, for example, to apply liquid layer 18, shown in Fig. 1A and to wet or re-wet patch 10 to maintain the patch 10 in a saturated state. According to one embodiment, dispenser pen 50 is provided with a removable cap 56, shown in Fig. 5 . According to one embodiment, cap 56 forms a friction fit over the end of dispenser pen 50 including orifice 52, closing orifice 52 to prevent liquid from unintentionally flowing out from reservoir 54. Fig. 6 shows cap 56 applied to pen 50. According to one embodiment, cap 56 includes a pintle (not shown) on its inside surface that is positioned to align with orifice 52 when the cap is fitted onto the dispenser. The pintle is shaped to extend into and plug orifice 52 to form a secure seal to prevent liquid from seeping out from reservoir 54.

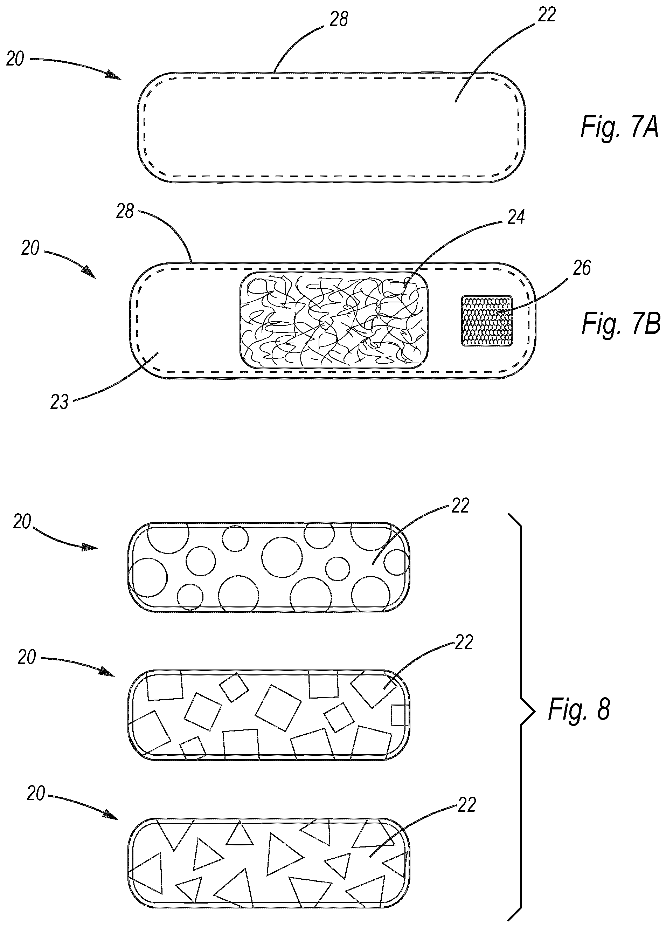

Figs. 7A and 7B show a simulated bandage 20 according to a further embodiment of the disclosure. Bandage 20 is formed from a flexible body 28. Body 28 has a top side 22, shown in Fig. 7A and a bottom side 23, shown in Fig. 7B . Top side 22 may include printing or other decoration to simulate the appearance of a real bandage. According to a further embodiment, shown in Fig. 8, top surface 22 may include decorative, colorful, or fanciful images.

Body 28 of bandage 20 is formed from a flexible material that can bend to at least partially conform to a three-dimensional shape such as the shape of a body part, such as a human limb. Body 28 may be formed from one or more of a natural or synthetic fabric, a polymer or elastomer sheet, leather, or other flexible material. According to a preferred embodiment, body 28 is formed from 2 mm thick white tricot fabric. According to one embodiment, the edges of body 28 include stitching to prevent the tricot fabric from fraying.

On the bottom surface 23 of bandage 20, as shown in Fig. 7B, a gripping portion 24 is provided. According to one embodiment, gripping portion 24 is formed from the same material that forms the "hook" portion of a "hook-and-loop" fastener. According to a preferred embodiment, gripping portion 24 is formed from nylon hook material. According to a more preferred embodiment, gripping portion 24 is formed from soft nylon and is selected to be safe in contact with human skin.

Bottom surface 23 of bandage 20 may also include retaining portion 26. According to one embodiment, retaining portion 26 is formed from "hook" material, such as the material forming gripping portion 24. The material forming retaining portion 26 is selected to removably engage with fibers of the fabric forming body 28 so that, when bandage 20 is wrapped around a body part, such as the user's finger, retaining portion 26 releasably connects with top surface 22 to temporarily hold bandage 20 onto the body part.

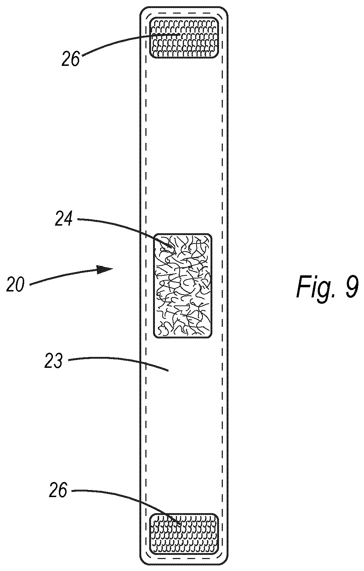

Fig. 9 shows the bottom side 23 of bandage 20 according to a further embodiment of the disclosure. In this embodiment, bandage 20 may be longer than in the previous embodiment. One or more gripping portions 24 and one or more retaining portions 26 are provided. As with the previous embodiment, retaining portions 26 interact with the top surface of the bandage to temporarily hold bandage 20 on the user's body. By providing a longer bandage 20, the embodiment in Fig. 9 can be affixed to a large body part, such as by being wrapped around an arm or leg.

The disclosure is not limited to bandages 20 that are rectangular. Other shapes are included within the scope of the disclosure, including, but not limited to round, square, oval, polygonal, star-shaped, or that are cut into a selected profile, for example, "Mickey Mouse Ears."

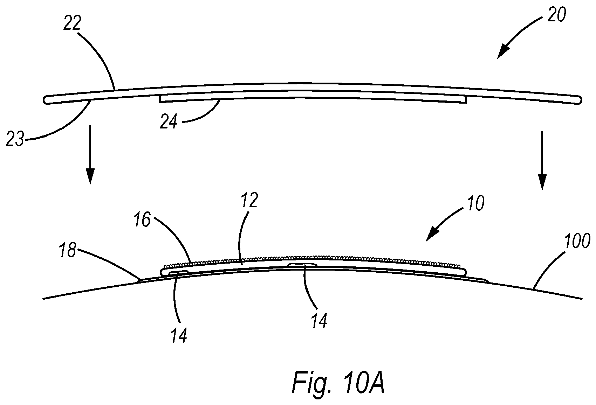

Figs. 10A to 10C show a patch 10 and bandage 20 according to a further embodiment of the disclosure and steps for applying and removing the patch. Fig. 10A shows patch 10 adhered to skin 100 by a liquid layer 18. As discussed above, when fabric layer 12 forming patch 10 is saturated, it becomes translucent, allowing the color of skin 100 to be visible through the fabric. Patch 10 includes simulated wound image 14, such as one shown in Figs. 2A to 2C . While patch 10 is saturated and adhered to skin 100, simulated wound image 14 creates the illusion that there is an actual wound on the skin.

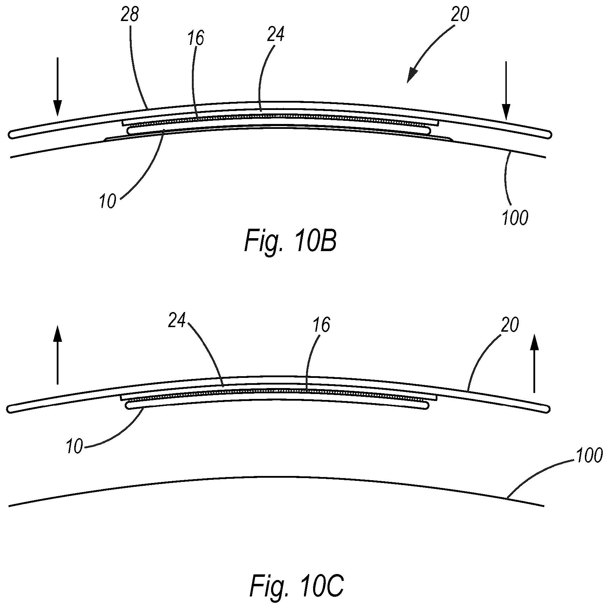

As shown in Fig. 10B, bandage 20 is placed on top of patch 10. Gripping portion 24 contacts the top surface of patch 10. Fibers 16 on the top of patch 10 engage with hook fasteners on gripping portion 24, forming a temporary bond between patch 10 and bandage 20. As shown in Fig. 10C, when bandage 20 is lifted away from skin 100, patch 10 adheres to bandage 20, lifting the patch away from the skin and giving the appearance that the wound has healed. Because the surface tension forces holding the saturated patch 10 against the skin 100 are relatively weak, bandage 20 easily lifts patch 10 from the skin.

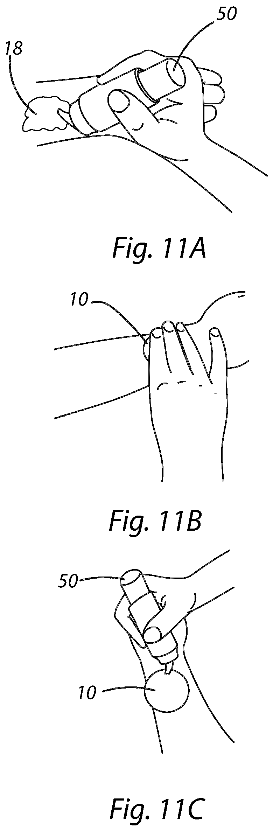

Figs. 11A to 11E show steps for applying and removing patch 10 to create a simulated wound and to give the appearance of having healed the wound. As shown in Fig. 11A, a user applies a layer of water 18 to the skin. According to one embodiment, water is applied using a dispenser, such as dispenser pen 50, discussed above. According to other embodiments, water is applied using a brush, a spray bottle, a fingertip, a sponge, and the like. According to another embodiment, water is applied by immersing the user's limb in a container of water, such as in a bathtub while a child is bathing.

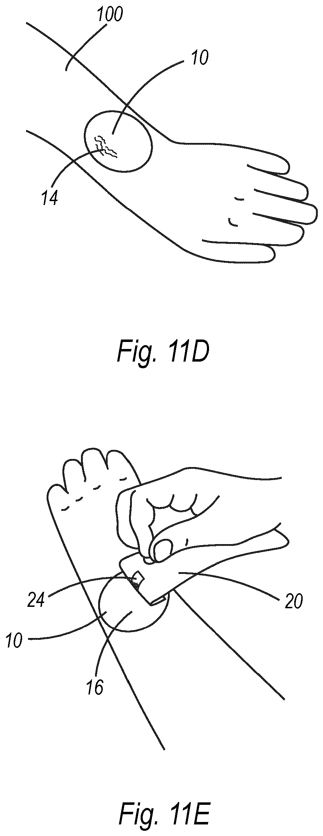

As shown in Fig. 11B, patch 10 is applied to the moistened skin. According to one embodiment, additional water may be applied to patch 10, as shown in Fig. 11C, to ensure that patch 10 is saturated so that the patch adheres to the skin and becomes translucent. As shown in Fig. 11D, saturated patch 10 adheres to the skin and displays simulated wound image 14 or other artwork surrounded by skin 100 surrounding the image. As shown in Fig. 11E, the user applies bandage 20 to patch 10. Gripper portion 24 of bandage 20 engages with gripping layer 16 of patch 10. When bandage 20 is pulled away from the skin, patch 10, along with the simulated wound image 14, revealing a "healed" wound.

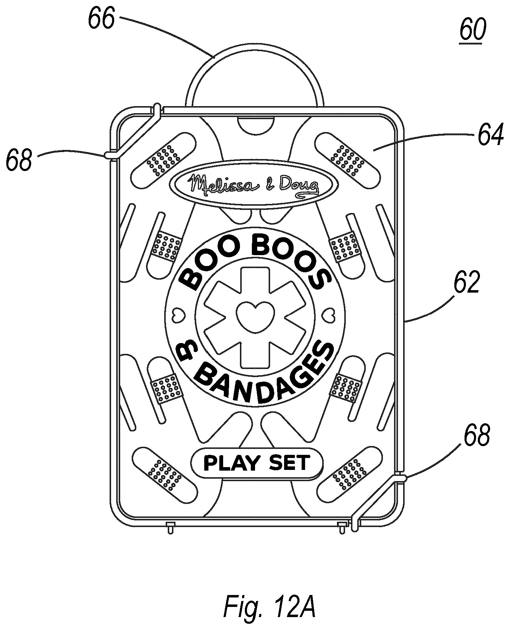

According to one embodiment of the disclosure, patches 10, bandages 20, and dispenser pen 50 are provided as components of kit 60. Fig. 12A shows the outside of kit 60 that includes housing 62 and lid 64 that form a convenient carrying case. Carrying handle 66 is provided. Handle 66 may be a loop of cord, a knob, a grip, a wooden handle, or other structure known in the field of the disclosure that facilitates carrying portable objects. According to the embodiment shown in Fig. 12A, lid 64 is fitted onto housing 62 by a clearance fit. One or more closures 68 connect with housing 62 and extend over the top surface of lid 64. Closures 68 may be elastic cords connected with housing 62 that are resiliently pulled over lid 64 to hold it to the housing. According to other embodiments, lid 64 is connected to housing 62 by one or more hinges so that the lid can swing away from housing 62 to expose the contents of the housing.

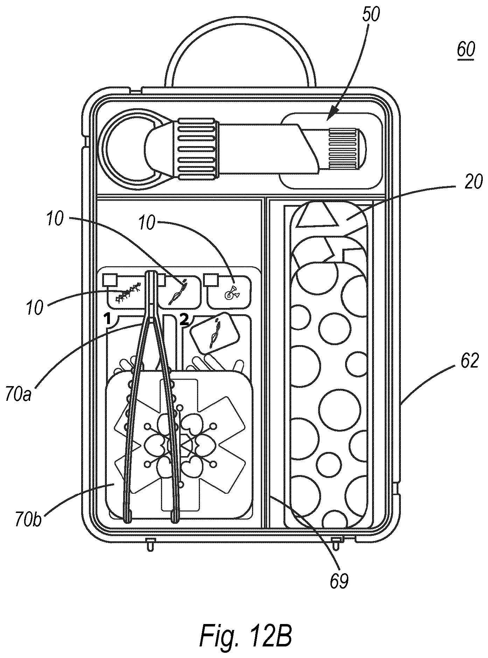

Fig. 12B shows kit 60 with lid 64 removed. Kit 60 includes one or more patches 10, one or more bandages 20, and a dispenser pen 50, such as those described above. Housing 62 may include one or more dividers 69 that define compartments to house components of the kit. Kit 60 includes a plurality of patches 10 in one such compartment. Patches 10 may have a variety of simulated wounds printed thereon as described above. Kit 60 includes a plurality of simulated bandages 20 held in another compartment. Kit 60 also includes dispenser pen 50 held in another compartment. According to some embodiment, kit 60 also includes other objects that relate to providing first aid, for example, tweezers 70a and a hot/cold pack 70b.

While the invention has been particularly shown and described with reference to exemplary embodiments thereof, the invention is not limited to these embodiments. It will be understood by those of ordinary skill in the art that various changes in form and details may be made therein without departing from the scope of the invention as defined by the following claims. Therefore, the description should not be construed as limiting the scope of the invention.

## Claims

1. A patch (10) adapted to display a simulated wound, the patch comprising:
a fabric layer (12) having a first translucency when dry and a second, greater translucency when saturated with a liquid; and
a simulated wound image (14) applied to the fabric layer (12), wherein, when the fabric layer (12) is applied to a skin (100) of a user and is saturated to achieve the second translucency, a color of the user's skin (100) is visible through the patch (10), and wherein the simulated wound image (14) is visible on the patch (10) to display the simulated wound image (14).

2. The patch (10) according to claim 1, further comprising a gripping layer (16) on a top surface of the fabric layer (12).

3. The patch (10) according to claim 2, wherein the gripping layer (16) comprises fibers that protrude from the upper surface of the patch (10), the fibers more particularly forming loops protruding from the upper surface.

4. The patch (10) according one of claims 1 to 3, wherein the fabric layer (12) comprises one or more of a woven textile, a non-woven textile, a natural textile, a synthetic textile, cotton, wool fabric, gauze, muslin, linen, polyethylene, or polypropylene and/or wherein the fabric layer (12) is white or a neutral color.

5. The patch (10) of claim 4, wherein the fabric layer (12) comprises non-woven polypropylene.

6. The patch (10) according to one of the preceding claims, wherein the simulated wound image (14) is applied to the fabric layer (12) by one or more of printing, screen printing, thermal transfer printing, and hand drawing.

7. The patch (10) according to one of the preceding claims, wherein the liquid comprises one or more of water, sea water, an aqueous dye solution, a non-aqueous material, mineral oil, petroleum jelly, baby oil, hand lotion, and sunscreen.

8. A kit (60) for creating a simulated wound comprising:
the patch (10) according to one of the preceding claims; and
a dispenser (50) adapted to deliver the liquid to the skin (100).

9. The kit (60) of claim 8, wherein the dispenser (50) comprises a reservoir (54) and a dispensing orifice (52) in fluid connection with the reservoir (54), wherein a limited flow of the liquid flows from the reservoir (54), through the orifice (52), and onto the user's skin (100), the dispenser (50) more particularly including a cap (56) removably connected with the dispenser (50) and adapted to close the orifice (52) to prevent the liquid from flowing from the reservoir (54).

10. A simulated bandage (20) configured to cooperate with the patch according to claim 2 or 3, comprising:
a thin, flexible body (28) having a bottom side (23), wherein the flexible body (28) is adapted to at least partially conform to a three-dimensional shape; and
a gripping portion (24) on at least a portion of the bottom side (23), said gripping portion (24) being configured to cooperate with the gripping layer (16) of the patch (10).

11. The simulated bandage (20) according to claim 10, wherein the gripping portion (24) comprises a plurality of hooks.

12. The simulated bandage (20) according to claim 11, wherein the gripping portion (24) comprises a hook component of a hook-and-loop fastener.

13. The simulated bandage (20) according to claim 10, 11 or 12, further comprising
a patch (10) adapted to create a simulated wound, the patch (10) comprising:
a fabric layer (12) having a first translucency when dry and a second, greater translucency when saturated with a liquid;
a simulated wound image (14) applied to the fabric layer (12), wherein, when the fabric layer (12) is placed on a skin (100) of a user and is saturated to achieve the second translucency, a color of the skin (100) is visible through the patch (10), and wherein the simulated wound image (14) is visible on the patch (10) when the patch (10) is saturated; and
a gripping layer (16) on a top surface of the fabric layer (12),
wherein the gripping portion (24) of the flexible body (28) engages with the gripping layer (16) of the patch (10), and wherein movement of the flexible body (28) away from the skin (100) pulls the patch (10) from the skin (100) to remove the simulated wound image (14).

14. The simulated bandage (20) according to claim 13, wherein the gripping portion (24) of the flexible body (28) comprises a plurality of hooks, wherein the gripping layer (16) of the patch (10) comprises a plurality of loops, and wherein engagement of the flexible body (28) with the patch (10) comprises interengagement of at least a portion of the hooks with at least a portion of the loops.

15. A method for creating a simulated wound using the patch according to one of the claims 1 to 7 and the simulated bandage according to one of the claims 10 to 14, comprising,
Providing the patch (10) (12);
providing the simulated bandage (20);
applying the liquid to a skin (100) of a user to create a liquid layer (18);
applying the patch (10) to the liquid layer (18), wherein liquid in the liquid layer (18) saturates the fabric layer (12) to achieve the second translucency, wherein a color of the skin (100) is visible through the patch (10), and wherein the simulated wound image (14) is visible on the patch (10) when the patch (10) is saturated with the liquid;
applying the simulated bandage (20) to the patch (10) with the gripping portion (24) in contact with fabric layer (12), wherein the gripping portion (24) engages with the fabric layer (12).

16. The method of claim 15, further comprising applying an additional amount of the liquid to the patch (10) after the step of applying the patch (10) to the liquid layer (18) and /or further comprising moving the simulated bandage (20) away from the skin (100), wherein the patch (10) remains engaged with the simulated bandage (20) and the patch (10) is removed from the skin (100).

## Patentansprüche

1. Pflaster (10), das angepasst ist, um eine simulierte Wunde anzuzeigen, das Pflaster umfassend:
eine Gewebeschicht (12), die eine erste Transluzenz in trockenem Zustand und eine zweite, größere Transluzenz aufweist, wenn sie mit einer Flüssigkeit gesättigt ist; und
ein simuliertes Wundbild (14), das auf die Gewebeschicht (12) aufgebracht ist, wobei, wenn die Gewebeschicht (12) auf eine Haut (100) eines Benutzers aufgebracht und zum Erreichen der zweiten Transluzenz gesättigt wird, eine Farbe der Haut (100) des Benutzers durch das Pflaster (10) sichtbar ist, und wobei das simulierte Wundbild (14) auf dem Pflaster (10) sichtbar ist, um das simulierte Wundbild (14) anzuzeigen.

2. Pflaster (10) nach Anspruch 1, ferner umfassend eine Greifschicht (16) auf einer Oberseite der Gewebeschicht (12).

3. Pflaster (10) nach Anspruch 2, wobei die Greifschicht (16) Fasern umfasst, die von der Oberseite des Pflasters (10) hervorstehen, wobei die Fasern insbesondere Schlaufen bilden, die von der Oberseite hervorstehen.

4. Pflaster (10) nach einem der Ansprüche 1 bis 3, wobei die Gewebeschicht (12) eines oder mehrere von einem gewebten Textil, einem Vliesstoff, einem Naturtextil, einem synthetischen Textil, Baumwolle, Wollstoff, Gaze, Musselin, Leinen, Polyethylen oder Polypropylen umfasst und/oder wobei die Gewebeschicht (12) weiß oder eine neutrale Farbe ist.

5. Pflaster (10) nach Anspruch 4, wobei die Gewebeschicht (12) Vlies-Polypropylen umfasst.

6. Pflaster (10) nach einem der vorherigen Ansprüche, wobei das simulierte Wundbild (14) durch eines oder mehrere von Drucken, Siebdruck, Thermotransferdruck und Handzeichnung auf die Gewebeschicht (12) aufgebracht ist.

7. Pflaster (10) nach einem der vorherigen Ansprüche, wobei die Flüssigkeit eines oder mehrere von Wasser, Meerwasser, einer wässrigen Farbstofflösung, einem nichtwässrigen Material, Mineralöl, Vaseline, Babyöl, Handlotion und Sonnenschutzmittel umfasst.

8. Kit (60) zum Erzeugen einer simulierten Wunde, umfassend:
das Pflaster (10) nach einem der vorherigen Ansprüche; und
einen Spender (50), der angepasst ist, um die Flüssigkeit zu der Haut (100) zuzuführen.

9. Kit (60) nach Anspruch 8, wobei der Spender (50) ein Reservoir (54) und eine Auslassöffnung (52) in Fluidverbindung mit dem Reservoir (54) umfasst, wobei ein begrenzter Fluss der Flüssigkeit aus dem Reservoir (54) durch die Öffnung (52) auf die Haut (100) des Benutzers fließt, wobei der Spender (50) insbesondere eine Kappe (56) umfasst, die lösbar mit dem Spender (50) verbunden ist und angepasst ist, um die Öffnung (52) zu verschließen, um zu verhindern, dass die Flüssigkeit aus dem Reservoir (54) fließt.

10. Simulierter Verband (20), der konfiguriert ist, um mit dem Pflaster nach Anspruch 2 oder 3 zusammenzuwirken, umfassend:
einen dünnen, flexiblen Körper (28) der eine Unterseite (23) aufweist, wobei der flexible Körper (28) angepasst ist, um sich zumindest teilweise an eine dreidimensionale Form anzupassen; und
einen Greifabschnitt (24) an zumindest einem Abschnitt der Unterseite (23), wobei der Greifabschnitt (24) konfiguriert ist, mit der Greifschicht (16) des Pflasters (10) zusammenzuwirken.

11. Simulierter Verband (20) nach Anspruch 10, wobei der Greifabschnitt (24) eine Vielzahl von Haken umfasst.

12. Simulierter Verband (20) nach Anspruch 11, wobei der Greifabschnitt (24) ein Hakenelement eines Klettverschlusses umfasst.

13. Simulierter Verband (20) nach Anspruch 10, 11 oder 12, ferner umfassend
ein Pflaster (10), das angepasst ist, um eine simulierte Wunde zu erzeugen, das Pflaster (10) umfassend:
eine Gewebeschicht (12), die eine erste Transluzenz in trockenem Zustand und eine zweite, größere Transluzenz aufweist, wenn sie mit einer Flüssigkeit gesättigt ist;
ein simuliertes Wundbild (14), das auf die Gewebeschicht (12) aufgebracht ist, wobei, wenn die Gewebeschicht (12) auf eine Haut (100) eines Benutzers aufgebracht und zum Erreichen der zweiten Transluzenz gesättigt wird, eine Farbe der Haut (100) durch das Pflaster (10) sichtbar ist, und wobei das simulierte Wundbild (14) auf dem Pflaster (10) sichtbar ist, wenn das Pflaster (10) gesättigt ist; und
eine Greifschicht (16) auf einer Oberseite der Gewebeschicht (12),
wobei der Greifabschnitt (24) des flexiblen Körpers (28) mit der Greifschicht (16) des Pflasters (10) eingreift und wobei eine Bewegung des flexiblen Körpers (28) weg von der Haut (100) das Pflaster (10) von der Haut (100) abzieht, um das simulierte Wundbild (14) zu entfernen.

14. Simulierter Verband (20) nach Anspruch 13, wobei der Greifabschnitt (24) des flexiblen Körpers (28) eine Vielzahl von Haken umfasst, wobei die Greifschicht (16) des Pflasters (10) eine Vielzahl von Schlaufen umfasst, und wobei der Eingriff des flexiblen Körpers (28) mit dem Pflaster (10) einen Eingriff zumindest eines Teils der Haken mit zumindest einem Teil der Schlaufen umfasst.

15. Verfahren zum Erzeugen einer simulierten Wunde unter Verwendung des Pflasters nach einem der Ansprüche 1 bis 7 und des simulierten Verbands nach einem der Ansprüche 10 bis 14, umfassend:
Bereitstellen des Pflasters (10) (12);
Bereitstellen des simulierten Verbands (20);
Auftragen der Flüssigkeit auf eine Haut (100) eines Benutzers, um eine Flüssigkeitsschicht (18) zu erzeugen;
Aufbringen des Pflasters (10) auf die Flüssigkeitsschicht (18), wobei Flüssigkeit in der Flüssigkeitsschicht (18) die Gewebeschicht (12) sättigt, um die zweite Transluzenz zu erreichen, wobei eine Farbe der Haut (100) durch das Pflaster (10) sichtbar ist, und wobei das simulierte Wundbild (14) auf dem Pflaster (10) sichtbar ist, wenn das Pflaster (10) mit der Flüssigkeit gesättigt ist;
Aufbringen des simulierten Verbands (20) auf das Pflaster (10) mit dem Greifabschnitt (24) in Kontakt mit der Gewebeschicht (12), wobei der Greifabschnitt (24) mit der Gewebeschicht (12) eingreift.

16. Verfahren nach Anspruch 15, ferner umfassend ein Auftragen einer zusätzlichen Menge der Flüssigkeit auf das Pflaster (10) nach dem Schritt eines Aufbringens des Pflasters (10) auf die Flüssigkeitsschicht (18) und/oder ferner umfassend ein Bewegen des simulierten Verbands (20) weg von der Haut (100), wobei das Pflaster (10) mit dem simulierten Verband (20) eingegriffen bleibt und das Pflaster (10) von der Haut (100) entfernt wird.

## Revendications

1. Patch (10) adapté pour représenter une blessure simulée, le patch comprenant :
une couche de tissu (12) présentant une première translucidité lorsqu'elle est sèche et une deuxième translucidité, supérieure, lorsqu'elle est saturée d'un liquide ; et
une image de blessure simulée (14) appliquée sur la couche de tissu (12), dans lequel, lorsque la couche de tissu (12) est appliquée sur une peau (100) d'un utilisateur et est saturée pour atteindre la deuxième translucidité, une couleur de la peau (100) de l'utilisateur est visible à travers le patch (10), et dans lequel l'image de blessure simulée (14) est visible sur le patch (10) pour représenter l'image de blessure simulée (14).

2. Patch (10) selon la revendication 1, comprenant en outre une couche de préhension (16) sur une surface supérieure de la couche de tissu (12).

3. Patch (10) selon la revendication 2, dans lequel la couche de préhension (16) comprend des fibres qui font saillie à partir de la surface supérieure du patch (10), les fibres formant plus particulièrement des boucles faisant saillie de la surface supérieure.

4. Patch (10) selon l'une des revendications 1 à 3, dans lequel la couche de tissu (12) comprend un ou plusieurs éléments parmi un textile tissé, un textile non tissé, un textile naturel, un textile synthétique, du coton, un tissu en laine, de la gaze, de la mousseline, du lin, du polyéthylène ou du polypropylène et/ou dans lequel la couche de tissu (12) est blanche ou de couleur neutre.

5. Patch (10) selon la revendication 4, dans lequel la couche de tissu (12) comprend du polypropylène non tissé.

6. Patch (10) selon l'une quelconque des revendications précédentes, dans lequel l'image de blessure simulée (14) est appliquée sur la couche de tissu (12) par un ou plusieurs éléments parmi l'impression, la sérigraphie, l'impression par transfert thermique et le dessin à la main.

7. Patch (10) selon l'une des revendications précédentes, dans lequel le liquide comprend un ou plusieurs éléments parmi l'eau, l'eau de mer, une solution aqueuse de colorant, un matériau non aqueux, de l'huile minérale, de la vaseline, de l'huile pour bébé, de la lotion pour les mains et de la crème solaire.

8. Kit (60) pour créer une blessure simulée comprenant :
le patch (10) selon l'une des revendications précédentes ; et
un distributeur (50) adapté à délivrer le liquide sur la peau (100).

9. Kit (60) selon la revendication 8, dans lequel le distributeur (50) comprend un réservoir (54) et un orifice de distribution (52) en connexion fluidique avec le réservoir (54), dans lequel un débit limité du liquide s'écoule du réservoir (54), à travers l'orifice (52), et sur la peau (100) de l'utilisateur, le distributeur (50) comprenant plus particulièrement un capuchon (56) raccordé de manière amovible au distributeur (50) et adapté pour fermer l'orifice (52) afin d'empêcher le liquide de s'écouler par le réservoir (54).

10. Pansement simulé (20) configuré pour coopérer avec le patch selon la revendication 2 ou 3, comprenant :
un corps mince, flexible (28) ayant un côté inférieur (23), dans lequel le corps flexible (28) est adapté pour se conformer au moins partiellement à une forme tridimensionnelle ; et
une partie de préhension (24) sur au moins une partie du côté inférieur (23), ladite partie de préhension (24) étant configurée pour coopérer avec la couche de préhension (16) du patch (10).

11. Pansement simulé (20) selon la revendication 10, dans lequel la partie de préhension (24) comprend une pluralité de crochets.

12. Pansement simulé (20) selon la revendication 11, dans lequel la partie de préhension (24) comprend un composant à crochets d'une fixation à crochets et à boucles.

13. Pansement simulé (20) selon la revendication 10, 11 ou 12, comprenant en outre
un patch (10) adapté pour créer une blessure simulée, le patch (10) comprenant :
une couche de tissu (12) présentant une première translucidité lorsqu'elle est sèche et une deuxième translucidité, supérieure, lorsqu'elle est saturée d'un liquide
une image de blessure simulée (14) appliquée sur la couche de tissu (12), dans lequel, lorsque la couche de tissu (12) est placée sur la peau (100) d'un utilisateur et est saturée pour atteindre la deuxième translucidité, une couleur de la peau (100) est visible à travers le patch (10), et dans lequel l'image de blessure simulée (14) est visible sur le patch (10) lorsque le patch (10) est saturé ; et
une couche de préhension (16) sur une surface supérieure de la couche de tissu (12),
dans lequel la partie de préhension (24) du corps flexible (28) se met en prise avec la couche de préhension (16) du patch (10), et dans lequel le mouvement du corps flexible (28) à l'opposé de la peau (100) arrache le patch (10) de la peau (100) pour retirer l'image de blessure simulée (14).

14. Pansement simulé (20) selon la revendication 13, dans lequel la partie de préhension (24) du corps flexible (28) comprend une pluralité de crochets, dans lequel la couche de préhension (16) du patch (10) comprend une pluralité de boucles, et dans lequel la mise en prise du corps flexible (28) avec le patch (10) comprend la mise en prise mutuelle d'au moins une partie des crochets avec au moins une partie des boucles.

15. Procédé de création d'une blessure simulée à l'aide du patch selon l'une des revendications 1 à 7 et du pansement simulé selon l'une des revendications 10 à 14, comprenant les faits de :
prévoir le patch (10) (12) ;
prévoir le pansement simulé (20) ;
appliquer le liquide sur la peau (100) d'un utilisateur afin de créer une couche de liquide (18) ;
appliquer le patch (10) sur la couche de liquide (18), dans lequel le liquide de la couche de liquide (18) sature la couche de tissu (12) pour atteindre la deuxième translucidité, dans lequel une couleur de la peau (100) est visible à travers le patch (10), et dans lequel l'image de blessure simulée (14) est visible sur le patch (10) lorsque le patch (10) est saturé de liquide ;
appliquer le pansement simulé (20) sur le patch (10) avec la partie de préhension (24) en contact avec la couche de tissu (12), dans lequel la partie de préhension (24) se met en prise avec la couche de tissu (12).

16. Procédé selon la revendication 15, comprenant en outre l'application d'une quantité supplémentaire de liquide sur le patch (10) après l'étape d'application du patch (10) sur la couche de liquide (18) et/ou comprenant en outre le déplacement du pansement simulé (20) à l'opposé de la peau (100), dans lequel le patch (10) reste mis en prise avec le pansement simulé (20) et le patch (10) est retiré de la peau (100).
